Europäisches Patentamt

European Patent Office

Office européen des brevets

(19)

(11) Publication number: **0 263 591**
**A2**

(12) **EUROPEAN PATENT APPLICATION**

(21) Application number: 87307693.9

(22) Date of filing: 01.09.87

(51) Int. Cl.⁴: **A61K 39/00** ,
//C12N15/00,C12N7/00

(30) Priority: 04.09.86 JP 208772/86

(43) Date of publication of application:
**13.04.88 Bulletin 88/15**

(84) Designated Contracting States:
**CH DE FR GB LI NL**

(71) Applicant: **DIRECTOR GENERAL OF NATIONAL INSTITUTE OF HEALTH**
**10-35, Kamiosaki-2-chome**
**Shinagawa-ku Tokyo(JP)**

(84) **CH DE FR GB LI NL**

(71) Applicant: **NIPPON ZEON CO., LTD.**
**6-1, 2-chome**
**Marunouchi Chiyoda-ku, Tokyo(JP)**

(84) **DE**

(71) Applicant: **CHIBA-KEN**
**1-1, Ichibacho**
**Chiba-shi(JP)**

(84) **CH DE FR GB LI NL**

(72) Inventor: **Kojima, Asato**
**28-16, Higashi-1-chome**
**Kuki-shi(JP)**
Inventor: **Yasuda, Atsushi**
**25-6, Shichirigahamahigashi-4-chome**
**Kamakura-shi(JP)**
Inventor: **Goto, Hiroyuki**
**873 Futoocho Kohoku-ku**
**Yokohama(JP)**
Inventor: **Saeki, Sakiko**
**11-3 Nakarokugo-2-chome**
**Ota-ku, Tokyo(JP)**
Inventor: **Kamogawa, Kouichi**
**2151-42, Kamariyacho**
**Kanazawa-ku Yokohama(JP)**
Inventor: **Morita, Michio**
**10-4, Chishirodaihigashi-1-chome**
**Chiba-shi(JP)**
Inventor: **Watanabe, Kuniko**
**8-12 Hakusan-2-chome**
**Abiko-shi(JP)**
Inventor: **Kobayashi, Hiroshige**
**268, Chinmatsudo-3-chome**
**Matsudo-shi(JP)**

EP 0 263 591 A2

(74) Representative: **Harrison, David Christopher et al**
**MEWBURN ELLIS & CO 2/3 Cursitor Street London EC4A 1BQ(GB)**

(54) **Recombinant vaccinia virus.**

(57) A process for producing a vaccine, characterized by growing in a host a recombinant vaccinia virus obtained by inserting DNA having promoter function and heterogeneous DNA which can be expressed under the control thereof, in close vicinity to each other into a non-essential DNA region of an attenuated Lister temperature-sensitive mutant vaccinia virus strain which has no high-temperature growability in rabbit kidney cells and has a small pock size on the chorioallantoic membrane of an embryonated egg.

# RECOMBINANT VACCINIA VIRUS

This invention relates to a recombinant vaccinia virus.

In recent years, there has been invented a method for constructing a recombinant vaccinia virus inserted heterogenous DNA therein and there has been proposed a method using as live vaccine recombinant vaccinia virus constructed by insertion of, for example, DNA coding for infections disease pathogen antigen as heterogenous DNA (e.g., USP 4,603,112, WO 84/02077, etc.).

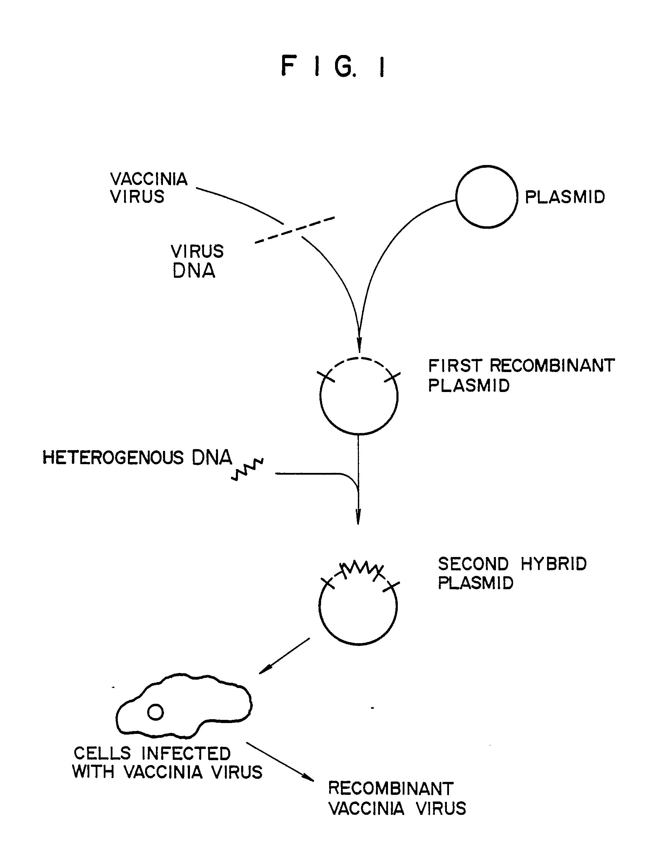

This method is practised according to, for example, the following procedure (see Fig. 1). First, suitable DNA is cut out of a DNA region non-essential to the growth of vaccinia virus by use of restriction enzymes, and inserted into a plasmid to prepare a first recombinant plasmid. Subsequently, heterogenous DNA is inserted into the vaccinia virus DNA portion of the recombinant plasmid to prepare a second recombinant plasmid.

On the other hand, animal cells previously infected with the vaccinia virus are prepared and the second recombinant plasmid is introduced into the animal cells, whereby homologous recombination between the vaccinia virus and the second recombinant plasmid is caused in the infected animal cells and a recombinant vaccinia virus having the heterogenous DNA introduced thereinto (e.g., the above-mentioned prior art references, Cell Technology Vol. 2, No. 9, p. 84 - 87 (1983), etc.).

According to this method, various heterogeneous DNAs can be inserted depending on purposes, and this method is useful as a novel method for producing live vaccine.

However, most of vaccinia viruses used in conventional method are of the WR strain (the abovementioned prior art references, Journal of Virology 49, 857-864 (1984), etc.), and since these vaccinia viruses have such strong postvaccinal complications that encephalitis or enicephalopathy as well as progressive vaccinia often occur, it has been difficult to put them into practical use from the viewpoint of safety.

Therefore, the present inventors studied in order to develop a safer live vaccine and consequently found that it was effective to use as vector an attenuated Lister temperature-sensitive mutant (i.e., an attenuated Lister temperature-sensitive mutant vaccinia virus strain which has no high-temperature growability in rabbit kidney cells and has a small pock size on the chorioallantoic membrane of an embryonated egg) in place of the WR strain, and the present inventors previously applied for a patent (Japanese Patent Application No. 18590/85).

However, although a recombinant obtained by this method is excellent in safety, it is insufficient in expression efficiency because the heterogeneous DNA inserted in the vaccinia virus genome is under the control of a promoter inherent in a non-essential DNA region of the vaccinia virus. Said recombinant has been disadvantageous in that particularly when it is inoculated into experimental mammals, immunization by a protein coded for by the heterogenous DNA is difficult to achieve.

## OBJECTS AND SUMMARY OF THE INVENTION

An object of this invention is to provide a vaccinia virus strain having a high expression efficiency by inserting DNA having promoter function and heterogenous DNA in close vicinity to each other into an attenuated Lister temperature-sensitive mutant.

Another object of this invention is to provide a process of recombination for preparing the above-mentioned vaccinia virus.

Further another object of this invention is to provide a process for producing a vaccine by growing the above-mentioned vaccinia virus strain.

Thus, according to this invention, there are provided a recombinant vaccinia virus obtained by inserting DNA having promoter function and heterogeneous DNA which can be expressed under the control thereof, in close vincinity to each other into a non-essential DNA region of an attenuated Lister temperature-sensitive mutant vaccinia virus strain which has no high-temperature growability in rabbit kidney cells and has a small pock size on the chorioallantoic membrane of an embryonated egg; a process for recombination for preparing said vaccinia virus; and a process for producing a vaccine.

BRIEF DESCRIPTION OF DRAWINGS

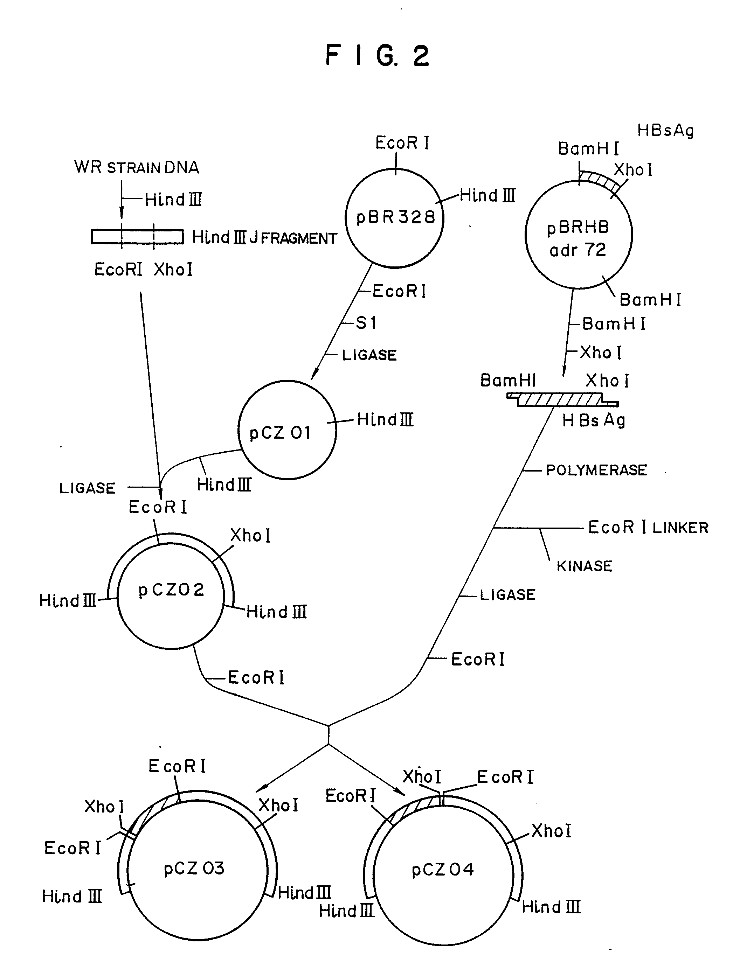

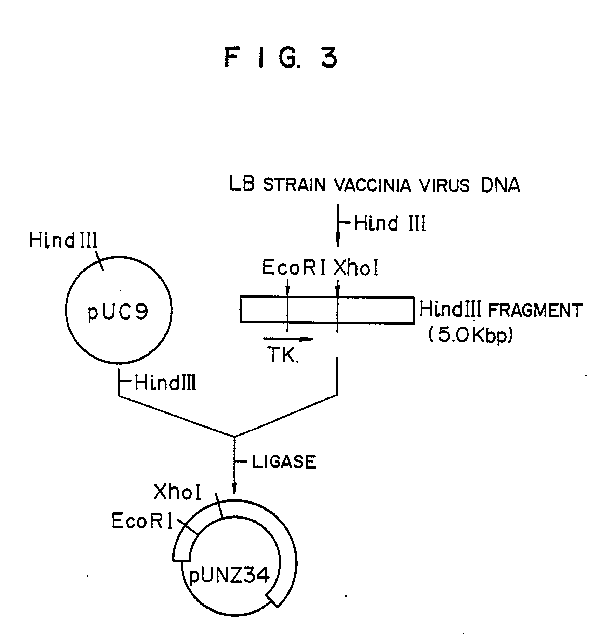

Fig. 1 shows an outline of a procedure for preparing a recombinant vaccinia virus. Fig. 2 shows the procedure carried out in Comparative Example 2. Figs. 3 to 6 show the procedures carried out in Example 1.

DESCRIPTION OF THE PREFERRED EMBODIMENTS

The vaccinia virus used for inserting thereinto heterogenous DNA is an attenuated temperature-sensitive mutant of a Lister strain virus, has a pock size on the chorioallantoic membrane of an embryonated egg of 3 mm or less, preferably 1 mm or less which is smaller than that of the original strain (i.e., Lister strain), and a shut-off temperature in rabbit kidney cells of 41°C or lower, preferably 40.5°C or lower.

The term "attenuated" used herein means that said mutant exhibits a greatly lower pathogenicity in the central nervous system of rabbits and monkeys than does the original strain (i.e. Lister strain) ane substantially no invasiveness caused by peripheral infection into the central nervous system of mice (see Example 4 of Japanese Patent Application Kokai (Laid-Open No. 44178/87). A paticularly preferable mutant is such that 6 days after inoculation of 5.8 $log_{10}TCID_{50}$/dose of the mutant into a Japanese white rabbit weighing 2.0 to 2.5 kg, the amount of virus recovered from 10% brain homogenate is 2 $log_{10}TCID_{50}$/ml or less, preferably 1 or less.

Such an attenuated temperature-sensitive mutant can be obtained, for example, by subculturing a Lister strain in rabbit kidney cells at 30°C, conducting plaque-cloning and then selecting a strain which shows very slight growth in Vero cells at 40°C. Further, it can be obtained by allowing the thus obtained strain to form pocks on the chorioallantoic membrane of an embryonated egg, and selecting a clone showing relatively small pocks (see USP 4,567,147).

Specific examples of the attenuated temperature-sensitive variant include LA strain, LB strain (CNTM-1-423), etc. prepared by the following method.

1) Preparation of LA strain

A Lister strain as original strain was subcultured in rabbit kidney cells over 36 generations at 30°C, and then plaque-cloned three times to isolate 50 clones. A temperature-sensitive mutant showing the worst growth at 40°C in Vero cells established from green monkey (Coropithecus aethiopus) kidney cells was selected from the 50 clones. Compared with the original strain, the temperature-sensitive mutant grew better in rabbit kidney cells but worse in rabbit central nervous system cells. In the central nervous system of a monkey, it exhibited a pathogenicity which was similar to that of DIs strain (an attenuated micropock mutant prepared from Dairen I strain); and extemely lower than that of the original strain. Since these facts had been confirmed, an inoculation test was carried out using a small number of subjects. As a result, it was found that said mutant would result in a light systemic reaction with a febrility ratio of 14% followed by somewhat slow formation of crust. Therefore, isolation of a clone showing a poor skin growth capacity was attempted. As a marker in the isolation of said clone, the size of pocks on the chorioallantoic membrane of an embryonated egg was employed. That is, the above-mentioned temperature-sensitive mutant was subcultured in rabbit kidney cells over six generations and then plaque-cloned twice to isolate a clone showing relatively small (2 to 3 mm) and uniform pocks on the chorioallantoic membrane of an embryonated egg, and the strain thus obtained was named LA strain. This strain had a shut-off temperature of 41°C and was much more attenuated that the original strain.

2) Preparation of LB strain

LA strain was further subcultured in rabbit kidney cells over additional three generations at 30°C to isolate a clone showing extremely small pocks (1 mm or less) on the chorioallantoic membrane of an embryonated egg, and the strain thus obtained was named LB strain. This strain has a shut-off temperature of 40.5°C and was still more attenuated than LA strain.

Comparison between properties of the Lister strain used as original strain and LB strain is as shown in Table 1.

Table 1: Comparison between Lister strain and LB strain

|  | Lister strain | LB strain |
|---|---|---|
| Shut-off temperature (in rabbit kidney cultured cells) | 41°C or above | 40.5°C |
| Plaque size (in rabbit kidney cultured cells) | large (3.5 mm) | medium (2.0 mm) |
| Pock size | large (4.0 mm) | small (0.9 mm) |
| Growability in chorioallantoic membrane of an embryonated egg | +++ | +++ |
| Growability in Vero cell | +++ | + (note 1) |
| Pathogenicity in the central nervous system |  |  |
| Rabbit (note 2) | +++ | + |
| Cynomolgus monkey (note 3) | yes (died) | no (survival) |
| Invasivaness by peripheral infection into central nervous system (note 4) |  |  |
| Mouse (cortisone acetate, subcutaneous inoculation) | yes | no |
| Mouse (untreated) | yes | no |
| Skin growth capacity |  |  |
| Rabbit | +++ | + |
| Human | ++ | + |

Note) 1: They differ from each other by about 2 $\log_{10}TCID_{50}$.

2: Evaluated from the amount of recovered virus six days after intracerabral inoculation of $10^{6.7}TCID_{50}$ of a virus.

3: Evaluated by intrathalmic inoculation of $10^{8.5}TCID_{50}$ of a virus.

As a method for inserting heterogeneous DNA having DNA with promoter function linked thereto into these vaccinia virus temperature-sensitive mutants, a conventional method may be employed. For example, the insertion can be achieved by utilizing a non-essential region, among the vaccinia virus DNA regions, whose loss has no fundamental influence on the growability of the resulting virus.

In detail, a first recombinant vector containing a part or the whole of the aforesaid non-essential DNA region is constructed, followed by constructing a second recombinant vector in which heterogeneous DNA having DNA with promoter function linked thereto has beein inserted in the non-essential DNA region inserted in the first recombinant vector, and the second recombinant vector is introduced into animal cells previously infected with the above-mentioned temperature-sensitive vaccinia mutant by conventional method such as the calcium phosphate method, whereby homologous recombination through said DNA region is caused in the infected animal cells to yield a recombinant vaccinia virus containing; as inserts DNA with promoter function and heterogenous DNA (e.g., WO 84/02077, Nature 302, 7, April 1983, p. 490 - 495, Proc. Natl. Acad. Sci. In USA 79, 7415-7419 (1982), etc.).

As the above-mentioned non-essential DNA region used in this invention, any non-essential DNA region may be used so long as it has no fundamental influence on the growability. Specific examples of the non-essential DNA region include, for example, a DNA region coding for thymidine kinase (TK), a DNA coding for hemagglutinin (HA), the Hind III F-fragment, F-fragment, M-fragment and N-fragment of WR strain vaccinia virus DNA (Virus 36 (1), 23 - 33 (1986)), and the DNA regions described in USP 4,603,112.

On the other hand, the vector used for constructing the first recombinant vector is not critical and any vector may be used so long as the non-essential DNA can be inserted thereinto. Specific examples of the vector include, for example, plasmids such as pBR 322, pBR 325, pBR 327, pBR 328, pUC 7, pUC 8, pUC 9, pUC 19 and the like, phages such as λ phage, M 13 phage and the like, and cosmids such as pHC 79 (Gene 11, 291 (1980)).

The first plasmid vector may be constructed by a conventional method. For example, vaccinia virus DNA is completely digested with suitable restriction enzymes, after which a DNA fragment corresponding to the non-essential region is separated and purified, and then ligated enzymatically with a vector cleaved by restriction enzymes which can produce in DNA the same cohesive end as that produced by the above restriction enzymes.

Whether the desired vector could successfully be obtained or not can be seen, for example, by a method which comprises transforming (or transducing) a micro-organism, e.g., Escherichia coli with a DNA mixture containing the recombinant vector obtained by the enzymatic ligation with the cleaved vector, and selecting a strain having the desired vector containing the non-essential region DNA inserted therein, from the transformed (or transduced) strains thus obtained.

In this invention, the second recombinant vector is constructed from the first recombinant vector thus obtained and heterogenous DNA having DNA with promoter function linked thereto.

As the DNA with promoter function used in this invention, DNA having any base sequence may be used, irrespective of synthetic and natural DNAs, so long as it functions effectively as promoter in a transcription system possessed by a vaccinia virus, and there are examplified, for example, such promoter sequences found in vaccinia virus genomes as exemplified in Journal of Virology Sept. 1984, p. 662 - 669, specifically promoter of a vaccinia virus gene coding for 7.5 K polypeptide, promoter of a vaccinia virus gene coding for 19 K polypeptide, promoter of a vaccinia virus gene coding for 42 K polypeptide, promoter of a vaccinia virus gene coding for thymidine kinase, promoter of a vaccinia virus gene coding for 28 K polypeptide, etc.

Although the heterogenous DNA used in not critical, it is preferably DNA coding for antigenic proteins of infectious disease pathogens (e.g., viruses, bacteria, parasites, protozoans, etc.). Specific examples of the heterogenous DNA include, for example, DNA regions coding for antigenic proteins of herpes virus, vesicular stomatitis virus, hepatitis B virus, hepatitis A virus, hepatitis non A non B agent, foot and mouth disease virus, poliomyelitis virus, rabies virus, Japanese encephalitis virus, cholera bacillus, salmonella, tetranus bacillus, Plasmodium, schistosome, etc. In addition to these DNAs coding for antigenic proteins, DNAs coding for useful porteins such as blood coagulation factors VIII and IX, etc. can also be used.

In this invention, the DNA having promoter function should be linked to the heterogenous DNA previously before constructing the second recombinant vector. This linkage may be conducted according to the method for constructing the first recombinant vector. For example, the DNA having promoter function is inserted into a vector, and then the heterogenous DNA is inserted thereinto downstream to the promoter site, in the correct reading direction, after which only necessary DNA region is excised from said vector with suitable restriction enzymes.

In this case, it is important to connect the DNA having promoter function and the heterogenous DNA in close vicinity to each other. Usually, the distance between the transcription initiation site present in the promoter site and the first translation initiation site present in the heterogenous DNA is adjusted to 200 base pairs or less, preferably 100 base pairs or less.

The second recombinant vector is prepared from the thus obtained heterogenous DNA having the DNA with promoter function directly linked thereto and the first recombinant vector. This preparation may also be carried out by a conventional method. For example, the first recombinant vector is cleaved wtih restriction enzymes having cleavage sites in the vacinnia virus non-essential DNA region inserted in the first recombinant vector, and both ends of the DNA fragments thus obtained are blunted using DNA polymerase, and heterogenous DNA fragment having DNA with promoter function directly directed therto whose both ends have been blunted similarly by DNA polymerase treatment are ligated enzymatically with the above-mentioned DNA fragments.

The desired second recombinant vector can be harvested in the same manner as for the first recombinant vector.

In this invention, the second recombinant vector is then introduced into animal cells previously infected with a vaccinia virus, thereby a recombinant vaccinia virus is formed.

As the animal cell used here, any cell may be used so long as the vaccinia virus can grow therein, and specific examples of the animal cell include, for example, TK-143 (derived from human osteosarcoma), FL (derived from human amnion), Hela (derived from carcinoma of human uterine cervix), KB (derived from carbinoma of human rhinopharynx), CJ-1 (derived from monkey kidney), BSC-1 (derived from monkey kidney), RK 13 (derived from rabbit kidney), L 929 (derived from mouse connective tissue), CE (chicken embryo) cell, CEF (chiken embryo fibroblast), etc.

As a method for introducing the recombinant vector into the animal cells, there may be employed a conventional method, for example, calcium phasphate method, liposome method, microinjection method, or the like.

Selection of the constructed recombinant vaccinia virus may also be carried out by a conventional method. For example, when the non-essential DNA region is a DNA region coding for thymidine kinase, the recombinant vaccinia virus can be obtained by culturing thymidine kinase-deficient cells infected with vaccinia virus which has undergone homologous recombination for 3 days under culture medium containing 25 μg/ml of 5-bromodeoxyuridine (BUdR), and selecting plaques formed by the recombinant vaccinia virus.

However, when a strain having a shut-off temperature of 40.5°C is used as Lister temperature-sensitive mutant vaccinia virus strain, it is very difficult to obtain a recombinant vaccinia virus, and plaques of the recombinant vaccinia virus can be selected only by conducting cultivation for 5 days or more by use of a culture medium having a lower BudR concentration.

As a process for producing a vaccine, a conventional process may be employed. For example, the recombinant vaccinia virus is inoculated into a suitable host, e.g., a small animal such as white rabbit and grown for a suitable period, after which the attenuated virus thus grown was isolated from the host and used as vaccine.

## EFFECTS OF THE INVENTION

The recombinant vaccinia virus thus obtained has, in vitro, a high expression activity of several hundred times or more that of the recombinant containing no DNA with promoter function inserted therein which the present inventors constructed previously (see Example 3) hereinafter described). Such an improving effect on expression activity is much more remarkable than that obtained in the case of using the WR strain as parent strain (see WO 84/02077). As compared with a recombinant obtained by using the WR strain as parent strain, the recombinant of this invention shows a much higher output of heterogenous protein per infected cell, though it has a lower virus infectivity titer.

Furthermore, the recombinant of this invention substantially equals in growability to a recombinant containing no DNA with promoter function inserted therein and is superior thereto in performance characteristics with regard to pathogenicity in rabbit central nervous system.

Thus, according to this invention, by inserting heterogenous DNA having DNA with promoter function linked directly thereto into a non-essential DNA region of a Lister temperature-sensitive mutant vaccinia virus strain which has no high-temperature growability in rabbit kidney cells and has a small pock size on the chorioallantoic membrane of an embryonated egg, there can be obtained a recombinant vaccinia virus which does not lose its growability even when inoculated into a mammal, can assure sufficient expression of the heterogenous DNA, permit production of antibody thereto, and is excellent in safety. Moreover, this recombinant vaccinia virus is advantageous in that because of the increase in the output of heterogenous protein per infected cell, the amount of cells used in tests, etc. can be reduced.

Examples

This invention is more concretely explained with reference to the following examples.

Comparative Example 1

(1) Preparation of a first vector (plasmid pCZ 02) containing vaccinia virus TK gene (see Fig. 2).

Using EcoR I, 5 μg of pBR 328 was digested, and then extracted with phenol-chloroform (1 : 1), and the cleaved PBR 328 was recovered by ethanol precipitation, after which both ends of the DNA fragments thus obtained were blunted by treatment with S1 enzyme. The thus treated DNA fragments were ligated to obtain a plasmid (pCZ 01) having no EcoR I cleavage site. pCZ 01 was selected by transforming competent Escrerichia coli strain C 600 cells with the ligated DNA, and selecting chloramphenicol-sensitive transformed strains.

Subsequently, 5 μg of plasmid pCZ 01 was treated with Hind III and extracted with phenol-chloroform, and aftrer ethanol precipitation, DNA was recovered. The phosphoric acid group at the 5'-terminal was removed by alkaline phosphatase treatment, and DNA was reextracted with phenol-chloroform and then recovered by ethanol precipitation. With 0.1 μg of purified vaccinia virus (WR strain) Hind III J fragment containing vaccinia virus thymidine kinase (TK) gene was ligated 0.5 μg of the cleaved pCA 01 DNA, and the recombinant plasmid thus obtained was named pCZ 02. The presence of the vacinia virus TK gene in pCZ 02 was confirmed by the following procedure. Competent Escherichia coli strain HB 101 cells were transformed with the ligated DNA and ampicilline-resistant, tetracycline-sensitive cells were selected. Then, plasmids were extracted from the cells by the method of Birnboin and Doly (Nucleic Acid Res., 7, 1513–(1979)) and digested with Hind III, after which whether a fragment having the same length as the original vaccinia virus Hind III J fragment was present or not was investigated by electrophoresis.

(2) Preparation of a second vector (plasmid pCZ 03) containing HBsAg gene (see Fig. 2).

Using BamH I and Xho I, 10 μg of plasmid pBRHBadr 72 containing a gene coding for heptatitis B surface antigen (HBsAg) (Nucleic Acid Res. Vol 11, No. 13, 4601-4610 (1983)) was digested, and then approx. 1.27 kb DNA fragments were separated by low-melting agarose electrophoresis (40 volts for 16 hours). Subsequently, the DNA fragments were identified by ethidium bromide dyeing, after which the gel was cut out, followed by extraction with phenol. Then, approx. 1.27 kb DNA fragments containing HBsAg gene were recovered by ehtanol precipitation. The recovered DNA fragments were dissolved in 10 μl of a buffer solution containing 10 mM Tris-HCl (pH 8.0) and 1 mM EDTA, treated with DNA polymerase to blunt their both ends, extracted with phenol-chloroform, and then recovered by ethanol precipitation.

On the other hand, the 5'-terminal of 1 μg of EcoR I linker was phosphorylated by use of 10 units of kinase in kination buffer at 37°C for 30 minutes, after which the previously prepared HBsAg DNA fragments were added and ligation was conducted at 12°C for 16 hours. The reaction mixture was treated with phenol-chloroform, and DNA was recovered by ethanol precipitation, further digested by addition of 20 units of EcoR I, and subjected to phenol-chloroform treatment, followed by ethanol precipitation.

On the other hand, pCZ 02 was linearized by treatment with EcoR I in an amount of 2 units per μg of DNA in a buffer solution at 37°C for 2 hours. The 5'-terminal of the linearized plasmid was dephospharylated with 0.1 unit of bovine small intestine alkaliphosphatase by allowing the linearized plasmid to stand in a buffer solution containing 50 mM Tris-HCl (pH 9.0), 1 mM MgCl₂, 0.1 mM ZnCl₂ and 1 mM spermidine. The buffer solution was extracted with an equal volume of phenol-chloroform, and DNA was recovered by ethanol precipitation. In a mixture of 66 mM Tris-HCl (pH 7.5), 6.6 mM MgCl₂, 10 mM DTT and 0.5 mM ATP, 0.1 μg of the linear plasmid thus obtained was treated at 12°C for 15 hours, and then ligated with 0.2 μg of the 1.27 kb EcoR I fragment containing HBsAg gene. The resulting plasmid was used for transforming Eshcrichia coli strain HB 101, and the transformed E. coli cells were cultured on LB culture media containing 1.5% of agar and 50 μg/ml of amplicillin at 37°C for 15 hours. The transformed E. coli cells grown on the agar were cultured in LB liquid medium containing 50 μg/ml of ampicillin at 37°C for 15 hours and purification was conducted by the method of Birnboim and Doly described above. In a buffer solution, 10% of each DNA sample was digested with 5 units of EcoR I at 37°C for 1 hour and screening was conducted by agarose electrophoresis to obtain 1.27 kb EcoR I fragments of plasmid DNAs.

Since the EcoR I hepatitis B virus DNA fragment of 1.27 kb can be inserted in the correct orientation or the reverse orientation in each plasmid, screening was conducted for the orientation of the inserted gene. It was conducted by digesting the plasmids derived from plasmid pCZ 02, with Xho I in a buffer solution for 1 hour, analyzing the resulting DNA fragments by agarose electrophoresis, and comparing their lengths. A plasmid in which HBsAg gene has been inserted in the correct orientation in relation to the vaccinia virus TK gene promoter was named pCZ 03, and a plasmid in which HBsAg gene had been inserted in the reverse orientation was named pCZ 04.

(3) Preparation of a recombinant vaccinia virus

RK-13 cells cultured in a Petri dish having a diameter of 6 cm were inoculated with an attenuated vaccinia virus strain (attenuated vaccinia strain LA, shut-off temperature in rabbit kidney cells 41°C, pock size on the chorioallantoic membrane of an embryonated egg 2 - 3 mm, a method for preparation from the original strain was described above) in an amount of 0.1 pfu/cell. In 2.2 ml of sterile water was dissolved 12 $\mu$g of plasmid pCZ 03 obtained by inserting HBsAg gene into vaccinia virus TK gene, and a DNA-calcium phosphate co-precipitate was prepared by the method of Hidaka et al. (Tampaku, Kakusan, Kaso (Protein, Nucleic Acid, Enzyme) 27, 340 (1985)). Forty-five minutes after the inoculation, 0.5 ml of the co-precipitate was dropped on the infected RK-13 cells. The Petri dish was allowed to stand in a 5% $CO_2$-incubator at 37°C for 30 minutes, and 4.5 ml of Eagle MEM containing 10% fetal calf serum was added. After 3 hours, the culture medium was replaced by a fresh liquid medium, and after cultivation for 48 hours, this culture medium was repeatedly subjected to freezing and thawing 3 times together with the cultured cells, followed by ultrasonication for 1 minute.

In order to confirm the insertion of HBsAg gene in the recombinant, TK-negative (TK⁻) (line 43) cells cultured in a Petri dish having diameter of 6 cm were inoculated with the above-mentioned plaque-forming virus, and after 45 minutes, the culture medium was overlaid with Eagle MEM containing 1% agar, 12% fetal calf serum and 25 $\mu$g/ml of BUdR. After cultivation for 3 days, infected cells were dyed with 0.01% Neutural red. The plaque formation percentage was about 0.005%. The agar plate was removed from the Petri dish and stored at 4°C. A sterile nylon membrane was pressed against the surface of the cells remaining in the bottom of the Petri dish to transfer virus, and treatment with 0.5 N NaOH for 10 minutes and then with 1 M Tris-HCl buffer for 5 minutes was repeated 3 times, followed by treatment with 0.5 M Tris-HCl buffer containing 1.5 NaCl for 5 minutes. The nylon membrane was saturated with 2 x SSC (1 x SSC = 0.15 M NaCl and 0.015 M $C_3H_4(OH)(COONa)_3$) and baked at 80°C for 2 hours. It was then treated with 4 x SET (0.6 M NaCl, 0.008 M Tris-HCl, 4 mM EDTA, pH 7.8) - 10 x Denhardt - 0.1% SDS at 68°C for 2 hours. Using 4 x SET - 10 x Denhardt - 0.1% SDS - 0.1% $Na_4P_2O_7$ -50 $\mu$g of denatured salmon sperm DNA/ml and hepatitis B DNA labeled with $^{32}P$ by nick-translation, hybridization was carried out at 68°C for 14 hours. After washing the nylon membrane and X-ray film were placed one upon another, and autoradiography was carried out. The presence of spots was confirmed, and the X-ray film was placed on the agar plate stored at 4°C. Plaques which coincides in position with the spots were identified as those due to a recombinant (rLA strain) containing HBsAg gene, and isolated by means of a sterile Pasteur pipet.

Comparative Example 2

A recombinant (rLB strain) containing HBsAg gene was obtained by carrying out an experiment according to Comparative Example 1, except that attenuated smallpox LB strain (shutt-off temperature in rabbit kideny cells: 40.5°C, pock size on the chorioallantoie membrane of an embryonated egg: about 0.9 mm, CNCM-I-423) was used as Lister temperature-sensitive mutant vaccinia virus, and that the method for selecting the recombinant vaccinia virus was changed as follows.

Method for selecting the recombinant:

TK-negative (TK⁻) (line 143) cells cultured in a Petri dish having a diameter of 6 cm were inoculated with the above-mentioned plaque-forming virus, and after 45 minutes, the culture medium was overlayed with Eagle MEM containing 1% agar, 12% fetal calf serum and 15 μg/ml of BUdR. After cultivation for 3 days, the Eagle MEM was overlaid with MEM haviang the same composition, and the cells were cultured for another 3 days. Then, infected cells were dyed with 0.01% Neutral red. Thereafter, they were treated in the same manner as in Comparative Example 1.

Example 1

(1) Preparation of a first recombinant vector (plasmid pUNZ 34) containing vaccinia virus TK gene (see Fig. 3)

Using Hind III, 5 μg of pUC 9 was digested, and then extracted with phenol-chloroform (1 : 1) and the cleaved pUC 9 was recovered by ethanol precipitation. The phosphoric acid group at the 5′-terminal was removed by alkaline phosphatase treatment, and DNA was reextracted with phenol-chloroform and then recovered by ethanol precipitation. With 0.1 μg of purified vaccinia virus (the above-mentioned LB strain) Hind III K fragment (about 5.0 kbp) was ligated 0.5 μg of the cleaved pUC 9 DNA, and the resulting recombinent plasmid was named pUNZ 34. The Hind III K fragment of Lister strain is known to correspond to Hind III J fragment of the WR strain (J. Gen. Virol. 45, 683 (1979)), and it is believed that TK gene is present in the Hind III K fragment.

The presence of the Hind III K fragment in pUNZ 34 was confirmed by transforming competent Escherichia colis train JM-103 cells, allowing the transformant to form colonies on agar plate of LB culture media containing 5-bromo-4-chloro-3-indolyl-β-D-galactopyranoside (0.003%), isopropyl-β-d-galactopyranoside (0.03 mM) and 40 μg/ml of ampicillin, and extracting DNA from white colonies in the same manner as in Comparative Example 1.

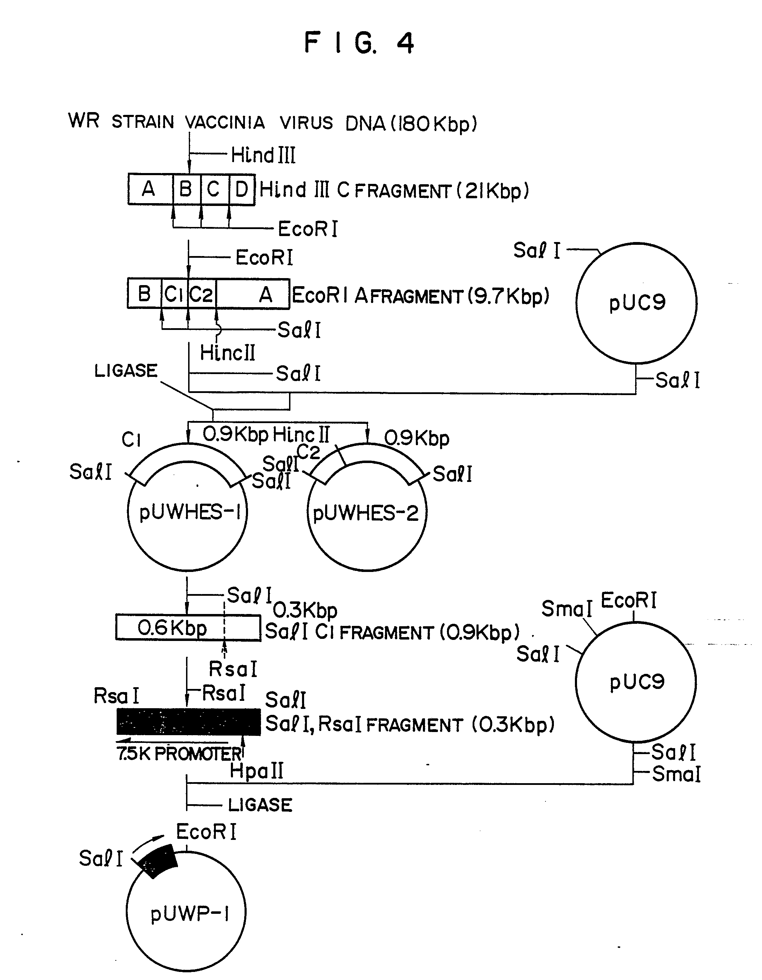

(2) Preparation of a plasmid (pUWP-1) containing a promoter of an early vaccinia virus gene coding for 7.5 k polypeptide (see Fig. 4)

Using restriction endonuclease Hind III, 5 μg of DNA (about 180 kbp) of a vaccinia virus (WR strain) was digested, and then approx. 21 kbp Hind III C fragments were separated by low-melting agarose electrophoresis (40 volts for 16 hours). Subsequently, the DNA fragments were identified by ethidium bromide dyeing, after which the gel was cut out and treated with phenol. Then, the Hind III C fragments were recovered by ethanol precipitation. Next, the Hind III C fragments were digested with restriction endonuclease EcoR I, and approx. 9.7 kbp EcoR I A fragments were separated under the same conditions as described above, extracted from gel in the manner described above, and then recovered by ethanol precipitation. Further, the EcoR I A fragments were digested with restriction endonuclease Sal I. On the other hand, 5 μg of pUC 9 was digested with Sal I, then extracted with phenol-chloroform (1 : 1), and recovered by ethanol precipitation. The fragment previously prepared by digestion with Sal I was ligated with the cleaved pUC 9, and the resulting recombinant plasmid containing a promoter region of the vaccinia virus coding for 7.5 K polypeptide was named pUWHES-1.

Four Sal I recognition sites are present in the above-mentioned EcoR I A fragment, and when this fragment is digested with Sal I, fragments containing the promoter region and fragments containing no promoter region are produced in substantially the same length (see Mass et al. Cell 125, 805 - 813 (1921). Therefore, a plasmid (pUWHES-2) having as an insert the fragment containing no promoter region is also produced at the same time. However, since Hinc II recognition site is present in this fragment, the two plasmids can be discriminanted from each other by taking advantage of the presence of said site.

In detail, pUWHES-1 was obtained by screening by transforming competent Escherichia coli strain JM 103 cells with the plasmid obtained,extracting plasmids in the same manner as in Comparative Example 1 (1), digesting the same with restriction endonuclease Hinc II, subjecting the digest to agarose electrophoresis, and selecting a plasmid which had given an approx. 0.9 kb fragment in the electrophoresis.

Subsequently, 5 μg of plasmid pUWHES-1 was digested with restriction endonuclease Sal I, and then 1 μg of a DNA fragment containing a 7.5 K promoter region of about 0.9 kbp was recovered by low-melting agarose gel electrophoresis. Using restriction endonuclease Rsa I, 1 μg of this DNA fragment was digested, after which 0.2 μg of a DNA fragment containing 7.5 K promoter region (the black portion in Fig. 4) was recovered by low-making agarose gel electrophoresis.

On the other hand, 0.5 μg of pCU 9 was digested with Sal I and Sma I, then extracted with phenol-chloroform (1 : 1), and recovered by ethanol precipitation. Subsequently, the DNA fragment thus obtained was ligated with the DNA fragment containing 7.5 K promoter region, and the resulting recombinant plasmid was named pUWP-1. The presence of the vaccinia virus 7.5 K promoter gene in pUWP-1 was confirmed from the following fact. Competent Escherichia coli strain JM 1-03 cells were transformed with this plasmid, and thereafter in the same manner as in Comparative Example 1 (1), extraction of plasmid, digestion thereof with Sal I and EcoR I and agarose electrophoresis of the digest were carried out, whereby an approx. 0.3 kbp fragment could be obtained.

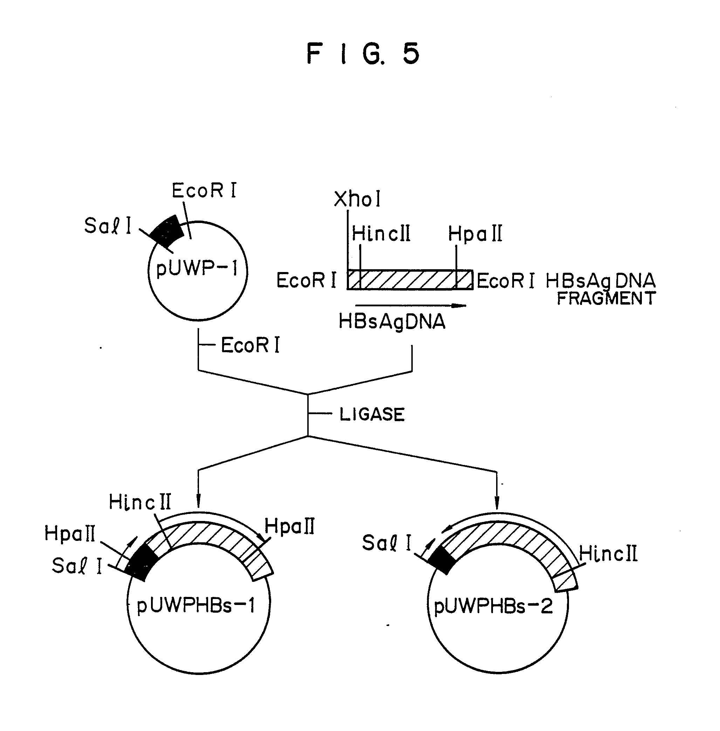

(3) Preparation of a plasmid (pUWPHBs-1) containing HBsAg gene directly linked to a promoter of an early vaccinia virus gene coding for 7.5 K polypeptide (see Fig. 5)

With EcoR I, 1.3 μg of plasmid pUWP-1 was treated, and extracted with phenol-chloroform, and after ethanol precipitation, DNA was recovered. The phosphoric acids group at the 5'-terminal was removed by alkaline phosphatase treatment, and thus treated DNA was reextracted with phenol-chloroform and then recovered by ethanol precipitation. With an EcoR I DNA fragment of about 1.27 kbp including a gene coding for hepatitis B surface antigen (HBSAg) (the same as used in Comparative Example 1) was ligated 1.3 μg of the cleaved uPWP-1 DNA.

Since the EcoR I hepatitis B virus DNA fragment (the shadowed portion in Fig. 5) can be inserted in the correct orientation or the reverse orientation in relation to the inserted promoter in each plasmid, screening was conducted for the orientation of the inserted gene. It was conducted by digesting the plasmids derived from plasmid pUWP-1, with Hinc II in a buffer solution, analyzing the resulting DNA fragments by agarose electrophoresis, and comparing their length. A plasmid in which HBsAg gene had been inserted in the correct orientation in relation to the vaccinia virus 7.5 K promoter gene was named pUWPHBs-1, and a plasmid in which HBsAg gene had been inserted in the reverse orientation was named pUWPHBs-22. The distance between the transcription initiation site in the promoter site and the translation initiation site in the EcoR I HNsAg DNA fragment both inserted in plasmid pUWPHNs-1 was about 60 bp.

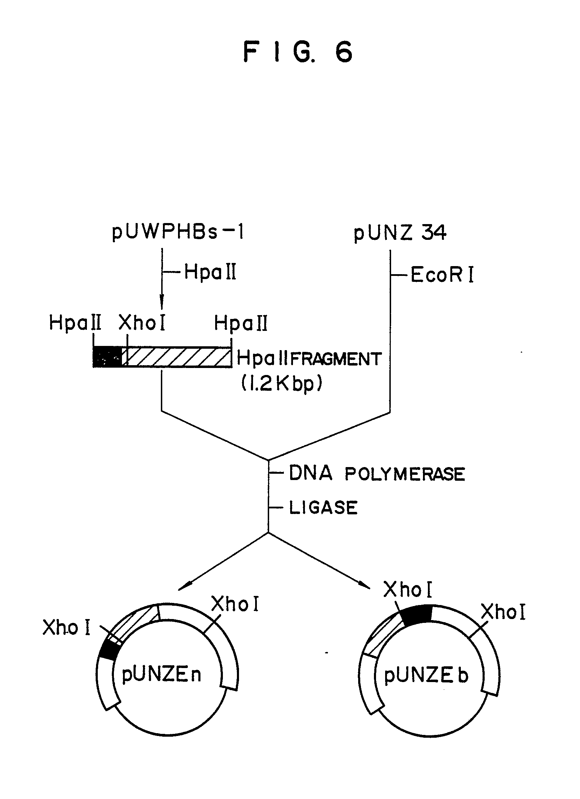

(4) Preparation of a second recombinant vector (plasmid pUNZen) containing HBsAg gene to which a promoter of an early vaccinia virus gene coding for 7.5 K polypeptide has been linked (see Fig. 6)

Using EcoR I, 5 μg of the plasmid pUNZ 34 obtained in (1) above was digested and then extracted with phenol-chloroform (1 : 1), and the cleaved pUNZ 34 was recovered by ethanol precipitation.

Subsequently, 10 μg of the plasmid pUWPHBs-1 prepared in (3) above was digested with Hpa II, and then approx. 1.2 kbp DNA fragments were separated by low-melting agarose electrophoresis (40 volts for 16 hours). Next, the DNA fragments were identified by ethidium bromide dyeing, and then the gel was cut out, followed by extraction with phenol. Then, approx. 1.2 kb DNA fragment, including vaccinia 7.5 K promoter gene and HBsAg gene were recovered by ethanol precipitation. The above-mentioned cleaved pUNZ 34 was added to these DNA fragments and their both ends were blunted by DNA polymerase treatment, followed by extraction with phenolchloroform and recovery by ethanol precipitation. The DNA fragments thus obtained wree ligated, and the resulting recombinant plasmid containing HBsAg having HBsAg gene directly linked thereto was named pUNZEn.

pUNZEn was identified as the desired plasmid by digesting the obtained plasmid with Xho I, analyzing the resulting DNA fragments by agarose electrophoresis, and comparing their length.

(5) Preparation of a recombinant vaccinia virus

A recombinant (rLA-P strain) containing the 7.5 K promoter gene and HBsAg gene was obtained by carrying out an experiment in the same manner as described in Comparative Example 1 (3), except that the plasmid pUNZen prepared in (4) above was used in place of plasmid pCZ 03.

Example 2

A recombinant (rLB-P strain) containing the 7.5 K promoter gene and HBsAg gene was obtained by carrying out an experiment in the same manner as described in Comparative Example 2, except that the plasmid pUNZEn prepared in Example 1 (4) was used in place of plasmid pCZ 03.

Example 3

Expression by a recombinant in cells

RK-13 cells (about 5 x $10^5$ cells) cultured in a short test tube was inoculated, at m.o.i. of 28.2 to 32.3, with each of the virus strains of this invention (rLA-P and rLB-P) and reference virus strains (rLA and rLB) containing no 7.5 K promoter inserted therein. After adsorption at 37°C for 1 hour, the viral solution was taken out and the cells were washed with Eagle MEM, after which Eagle MEM containing 2% calf serum was added.

In order to determine HBsAg and the vaccinia virus infectivity titer at the time of HBsAg production. The culture supernatant cell extract were collected at 24 hours an 48 hours of cultivation. The cell extract was obtained by collecting the culture supernatant in the short test tube, adding an equal volume of Eagle MEM containing 2% calf serum to the residue, and repeating freez-thawing twice.

HBsAG was determined for each culture supernatant and each cell extract by enzyme immunoassay (EIA method, DAINABOT Co., Ltd.; Anzyme), and expressed in terms of value of absorbance at a wavelength of 492 nm ($OD_{492}$).

The vaccinia virus titer was determined in the following manner. The culture supernatant and the cell extract at each collection time were diluted decimally with Eagle MEM, and 0.1 ml of each dilution was inoculated into RK-13 cells cultured in 4 short test tubes, to be subjected to adsorption thereon at 37°C for 1 hour. After the adsorption, the viral solution was taken out, and 0.5 ml of Eagle MEM containing 2% calf serum was added, after which the resulting mixture was subjected to roller culture at 37°C for 7 days. The case where CPE appeared was defined as infection and $TCID_{50}$ml was calculated.

Consequently, as compared with rLA or rLB virus containing no 7.5 K promoter inserted therein, rLA-P or rLB-P virus, i.e., the recombinants of this invention showed a several hundred times higher HBSAg output at either 24 hours or 48 hours or cultivation for either the culture supernatant or the cell extract, and showed a greatly improved in vitro HBsAg expression percentage. The virus infectivity titers at the time of HBsAg determination at the above two points of time were $10^{5.0}$-$^{8.0}$ $TCID_{50}$/ml for all the virus strains and were not appreciably different in all the cases.

Table 2: HBsAg production by recombinants

| Strain tested | m.o.i Virus strain (pfu/cell) | Cultivation time | | | |
|---|---|---|---|---|---|
| | | 24 hours | | 48 hours | |
| | | Culture supernatant | Cell extract | Culture supernatant | Cell extract |
| **Strains of the invention** | rLA-P 28.2 | 0.4629x80 (6.0)* | 0.5055x80 (7.75) | 0.8519x160 (6.25) | 0.6819x80 (8.0) |
| | rLB-P 32.3 | 0.8194x40 (5.50) | 0.4970x80 (5.25) | 0.8863x80 (5.0) | 0.9930x80 (7.5) |
| **Reference strains** | rLA 28.2 | 0.0867 (5.25) | 0.0612 (7.51) | NM (6.50) | NM (7.75) |
| | rLB 30.3 | 0.1578 (5.75) | 0.0517 (7.25) | NM (6.25) | NM (7.75) |

\*: the upper row; HBsAg output ($OD_{492}$) per about $5 \times 10^5$ cells, cutoff value 0.084, the lower row; virus infectivity titer (Log $TCID_{50}$/ml)

NM: Not measured

Example 4

Inoculation of a vaccinia virus recombinant into a rabbit

$5 \times 10^8 TCID_{50}$ of each of vaccinia virus recombinant prepared in Examples or Comparative Example above or the parent strain thereof was inoculated into a Japanese white rabbit intravenously in the ear (iv) or subcutaneously on the back at separate 7 positions (id) to infect the rabbit therewith. After the inoculation, blood was drawn from the ear every other day unitl the 14th day and its serum was separated and then frozen and stored at -20°C.

No rabbit thus treated showed any abnormality during observation for the 14 days.

The frozen serum was thawed by allowing the same to stand at room temperature and the antibody titer against HBsAg was determined by enzyme immunoassay (AUSAB EIA. Abott Laboratories, the amount of sample 0.2 ml). The results obtained are shown in Table 3.

The antibody titer in Table 3 is expressed in terms of a degree of dilution at which each serum became positive when serially diluted and then tested.

Table 3: Anti-HBsAg antibody titer in rabbits infected with a recombinant vaccinia virus

| Strains | Reference strains | | Strains of the invention | | | |
|---|---|---|---|---|---|---|
| Days after vaccination | LB (iv) | rLB (iv) | rLA-P (iv) | rLA-P (id) | rLB-P (iv) | rLB-P (id) |
| 2 | <1 | <1 | <1 | <1 | <1 | <1 |
| 4 | <1 | <1 | 10 | 40 | 10 | 10 |
| 6 | <1 | <1 | >400 | >400 | 100 | 10 |
| 8 | <1 | 2 | >400 | >400 | >400 | 40 |
| 10 | <1 | <1 | >400 | >400 | 100 | 100 |
| 12 | <1 | <1 | >400 | >400 | 100 | 100 |
| 14 | <1 | <1 | >400 | >400 | 100 | 100 |

From the results it can be seen that the recombinants of this invention have a greatly improved ability to induce antibody production in vivo, as compared with the parent strain (LB strain) and the recombinant (rLB strain) obtained in Comparative Example 2.

Example 5

Reinoculation of a vaccinia virus recombinant into a rabbit

$5 \times 10^8$ $TCID_{50}$ of vaccinia virus recombinant rLA-P was inoculated into a Japanese white rabbit subcutaneously. 7 Days after the inoculation, the blood sample was taken to determine the antibody titer against HBsAg. The titer was 600 m I.U. The titer decreased with a laspe of the time and showed 21 m I.U. 16 months after inoculation.

In order to determine the change in the titer against HBsAg was additionally inoculated $5 \times 10^8$ $TCID_5$ of the recombinant rLA-P to the same rabbit. The titer 7 days after the inoculation was 594 m I.U.; that is, the rise in the titer which is almost the same one as that of the first inoculation was observed.

As shown above, because the present recombinant has a potency of causing immunity again, it can be understood that the present recombinant is a very promising material as a live vaccine.

The antibody titer agained HBsAg (m.I.U./ml) was determined according to RIA pallarel line determination method, using HBIG (10 I.U./ml) as a control.

Example 6

Growth of vaccinia virus recombinant in a rabbit body

The sera obtained in Example 4 were subjected to a hemagglutination inhibition (HI) test (Gakuyukai, National Institute of Health "Servey of Virus Experimentation" 2nd ed. p. 217~, Maruzen Co., Ltd. (June 1973)), whereby the antibody titer against vaccinia virus was investigated. The test was carried out by preparing serial 2-fold dilutions of each serum (namely, diluting each serum in $2^n$ times), and the maximum dilution of serum as which the serum exhibited inhibitory action on hemagglutination was expressed in terms of the value on n. The results obtained as shown in Table 4.

Table 4

| Strains / Days after vaccination | Reference strains | | Strain of the invention | |
|---|---|---|---|---|
| | LB (id) | rLB (id) | rLA-P (id) | rLA-P (id) |
| 2 | 2 | 2 | 3 | 3 |
| 4 | 5 | 4 | 3 | 3 |
| 6 | 5 | 6 | 4 | 8 |
| 8 | 5 | 6 | 5 | 7 |
| 10 | 7 | 6 | 7 | 7 |
| 12 | 7 | 6 | 5 | 7 |
| 14 | 7 | 6 | 5 | 7 |

From the results, it can be seen that the recombinants of this invention are substantially equal in growability to the parent strain in a rabbit body.

Example 6

Test for pathogenicity in rabbit central nervous system

An inracerebral inoculation test were carried out on rabbits in order to compare the pathogenicity in the central nervous system of the recombinants of this invention (rLA-P and rLB-P) with that of the recombinants containing no 7.5 K promoter inserted therein or the WR strain.

Each healthy Japanese white rabbit weighting 2.0 to 2.5 kg was inoculated intracerebally with 0.25 ml of a viral solution of $10^{6.8}$ TCID$_{50}$ of each virus strain. After clinical observation for 6 days, surviving rabbits were killed, and the virus was recovered from the brain and subjected to a pathologic and histologic examination. The results obtained are shown in Table 5.

Consequently, the recombinants of this invention (rLA-P and rLB-P) showed similar recovery of virus and pathogenicity in the central nervous system to those of rLA and rLB, but showed a lower recovery of virus and a much lower pathogenicity in central nervous system as comapred with the WR strain. These results suggest that the recombinant rLA-P and rLB-P, like their respective parent strains, are attenuated viruses.

The pathogenesis mechanism of postvaccinal encephalopathy and encephalitis has theretofore been explained by the assumption that vaccinia virus invade the brain and grows therein to cause inflammatory changes in meninges and plexus choroideus. The animal experiment described above is considered one model system for studying the pathogenicity in the central nervous system of virus, and the recombinants of this invention having only low pathogenicity in the central nervous system are considered highly safe viruses and will be applicable as live vaccines.

Table 5

| Strain tested | Virus strain | Attack rate | Mortality | Recovery* of virus ($Log_{10}$ $TCID_{50}$/ml) | Inflammation** in meninges and plexus choroideus | Parenchymatous lesion |
|---|---|---|---|---|---|---|
| Strains of the invention | rLA-P | 0/2 | 0/2 | <0.5 5.0 | +++ +++ | + + |
| | rLB-P | 0/2 | 0/2 | <0.5 0.75 | +++ ++ | + + |
| Reference strains | rLa | 0/2 | 0/2 | <0.5 4.25 | +++ +++ | ++ ++ |
| | rLB | 0/2 | 0/2 | <0.5 <0.5 | +++ +++ | ++ + |
| | WR | 2/2 | 1/2 | 6.0 6.75 | ++++ ++++ | +++ +++ |

*: Viruses infectivity titer of a 10% homogenate

**: +; slight, ++; medium, +++; medium to serious, ++++ serious lesion

0 263 591

## Claims

1. A process for producing a vaccine including growing in a host a recombinant vaccinia virus obtained by inserting DNA having promoter function and heterogeneous DNA which can be expressed under the control thereof, in close vicinity to each other into a non-essential DNA region of an attenuated Lister temperature-sensitive mutant vaccinia virus strain.

2. A process according to claim 1 wherein the strain is not growable at high temperatures in rabbit kidney cells and has a small pock size on the chorioallantoic membrane of an embryonated egg.

3. A process according to claim 1 wherein the strain is one having a pock size on the chorioallantoic membrane of an embryonated egg of 3mm or less, and a shut-off temperature in rabbit kidney cells of 41°C or lower.

4. A process according to claim 1, claim 2 or claim 3 wherein the host is a white rabbit.

F I G. I

# F I G. 2

# F I G. 3

LB STRAIN VACCINIA VIRUS DNA

├─Hind III

HindIII FRAGMENT
(5.0 Kbp)

EcoRI XhoI

TK.

HindIII

pUC9

├─HindIII

─ LIGASE

XhoI

EcoRI

pUNZ34

# F I G. 4

# F I G. 5

# F I G. 6